(19) Europäisches Patentamt
European Patent Office
Office européen des brevets

(11) Veröffentlichungsnummer: **0 145 662**
**B1**

# (12) EUROPÄISCHE PATENTSCHRIFT

(45) Veröffentlichungstag der Patentschrift: **29.04.87**

(51) Int. Cl.⁴: **C 07 C 157/14**, A 01 N 47/42

(21) Anmeldenummer: **84810588.8**

(22) Anmeldetag: **03.12.84**

## (54) Isothioharnstoffe.

(30) Priorität: **08.12.83 CH 6572/83**

(43) Veröffentlichungstag der Anmeldung:
**19.06.85 Patentblatt 85/25**

(45) Bekanntmachung des Hinweises auf die Patenterteilung:
**29.04.87 Patentblatt 87/18**

(84) Benannte Vertragsstaaten:
**AT BE CH DE FR GB IT LI NL**

(56) Entgegenhaltungen:
**EP-A-0 075 205**
**GB-A-2 039 885**

(73) Patentinhaber: **CIBA-GEIGY AG, Klybeckstrasse 141, CH-4002 Basel (CH)**

(72) Erfinder: **Drabek, Jozef, Dr., Benkenstrasse 12, CH-4104 Oberwil (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 145 662 B1

LIBER, STOCKHOLM 1987

**Beschreibung**

Die vorliegende Erfindung betrifft neuartige substituierte N-Phenyl-N'-acyl-isothioharnstoffe, Verfahren zu ihrer Herstellung und ihre Verwendung in der Schädlingsbekämpfung.

Die erfindungsgemässen neuen Verbindungen entsprechen der Formel

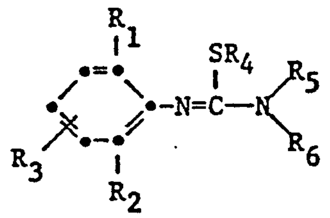

(I),

worin

$R_1$ $C_1$-$C_5$-Alkyl;

$R_2$ Wasserstoff oder $C_1$-$C_5$-Alkyl;

$R_3$ Wasserstoff Halogen $C_1$-$C_5$-Alkyl, Phenoxy oder durch 1 oder 2 Halogenatome, $C_1$-$C_5$-Alkyl- oder Trifluormethylreste substituiertes Phenoxy;

$R_4$ $C_1$-$C_5$-Alkyl, Allyl oder Propargyl;

$R_5$ $C_1$-$C_5$-Alkyl, $C_3$-$C_6$-Cycloalkyl, Allyl oder Propargyl; und

$R_6$ einen der Reste

-CO-$R_7$, -CO-CO-$R_8$, -$SO_2$-$R_7$ oder -CO-O$R_7$ bedeuten, wobei

$R_7$ $C_1$-$C_5$-Alkyl, Phenyl oder durch 1 oder 2 Halogenatome oder Methylreste substituiertes Phenyl, und

$R_8$ $C_1$-$C_{10}$ -Alkoxy oder Dialkylamino mit $C_1$-$C_5$-Alkylgruppen bedeuten.

Unter Halogen ist dabei Fluor, Chlor, Brom oder Jod, insbesondere aber Chlor, zu verstehen.

Die bei $R_1$ bis $R_8$ in Frage kommenden Alkyl- und Alkoxygruppen und -Substituenten können geradkettig oder verzweigt sein. Beispiele solcher Gruppen sind dementsprechend u.a. Methyl, Methoxy, Äthyl, Propyl, Isopropyl, n-, i-, sek.-, tert.-Butyl, n-Pentyl, n-Hexyl, n-Heptyl, n-Oktyl, n-Nonyl, n-Decyl und deren Isomere.

Cyclopropyl und Cyclohexyl sind die bevorzugten Cycloalkylgruppen bei $R_5$.

Erfindungsgemäss bevorzugt sind Verbindungen der Formel I, worin

$R_1$ $C_1$-$C_4$-Alkyl;

$R_2$ Wasserstoff oder $C_1$-$C_5$-Alkyl;

$R_3$ Wasserstoff, Fluor, Chlor, $C_1$-$C_3$-Alkyl, Phenyl, oder durch 1 oder 2 Fluor- oder Chloratome, Methyl-, Äthyl- oder Trifluoremethylreste substiuiertes Phenoxy;

$R_4$ $C_1$-$C_3$-Alkyl, Allyl oder Propargyl;

$R_5$ $C_2$-$C_5$-Alkyl, $C_3$-$C_6$-Cycloalkyl, Allyl oder Propargyl; und

$R_6$ einen der Reste

-CO-$R_7$, -CO-CO-$R_8$, -$SO_2$-$R_7$ oder -CO-O$R_7$, bedeuten, wobei

$R_7$ $C_1$-$C_4$-Alkyl, Phenyl oder durch 1 oder 2 Fluor- oder Chloratome oder Methylreste substituiertes Phenyl, und

$R_8$ $C_1$-$C_4$-Alkoxy oder Dialkylamino mit $C_1$-$C_3$-Alkylgruppen bedeuten.

Hervorzuheben sind weiterhin Verbindungen der Formel I, worin

$R_1$ $C_1$-$C_4$-Alkyl;

$R_2$ $C_1$-$C_5$-Alkyl;

$R_3$ Wasserstoff, Fluor, Chlor, Methyl, Phenoxy, durch ein Fluor- oder Chloratom einen Methyl- oder Trifluormethylrest substituiertes Phenoxy;

$R_4$ $C_1$-$C_3$-Alkyl, Allyl oder Propargyl;

$R_5$ $C_2$-$C_5$-Alkyl, Cyclopropyl, Cyclohexyl, Alkyl oder Propargyl; und

$R_6$ einen der Reste

-CO-$R_7$ oder -CO-CO-$R_8$ bedeuten, wobei

$R_7$ Methyl, Äthyl, Phenyl oder durch ein oder zwei Fluor- oder Chloratome substituiertes Phenyl; und

$R_8$ Methoxy, Äthoxy oder Dialkylamino mit Methyl- oder Äthylgruppen bedeuten.

Wegen ihrer biologischen, insbesondere insektiziden Wirkung, hervorzuheben, sind diejenigen Verbindungen der Formel I, worin

$R_1$ $C_1$-$C_3$-Alkyl;

$R_2$ $C_2$-$C_5$-Alkyl;

$R_3$ Wasserstoff oder 4-Phenoxy;

$R_4$ $C_1$-$C_3$-Alkyl, Allyl oder Propargyl;

$R_5$ $C_2$-$C_5$-Alkyl;

$R_6$ einen der Reste

-CO-$R_7$ oder -CO-CO-$R_8$ bedeuten, wobei

$R_7$ Methyl, Äthyl, Phenyl oder Difluorphenyl und

$R_8$ Methoxy, Äthoxy, Dimethylamino oder Diäthylamino bedeuten, und insbesondere solche Verbindungen

der Formel I, worin
$R_1$ $C_1$-$C_3$-Alkyl;
$R_2$ $C_2$-$C_4$-Alkyl;
$R_3$ Wasserstoff oder 4-Phenoxy;
$R_4$ Methyl;
$R_5$ $C_1$-$C_4$-Alkyl; und
$R_6$ einen der Reste $-CO-CH_3$ odel $-CO-CO-OC_2H_5$ bedeuten.

Die Verbindungen der Formel I werden analog bekannten Arbeitsweisen (vgl. z.B. US-Patentschrift 4,357,351) hergestellt, indem man einen Isothioharnstoff der Formel

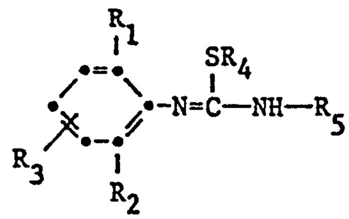

(II)

mit einem Halogenid der Formel III

X - $R_6$ (III)

umsetzt, wobei in den Formeln II und III $R_1$ bis $R_8$ die unter Formel vorstehend bereits angegebenen Bedeutungen haben und X für ein Halogenatom, insbesondere ein Chloratom, steht.

Das zu den erfindungsgemässen Verbindungen der Formel I führende Verfahren wird zweckmässig bei einer Temperatur zwischen -30 und 100°C, vorzugsweise zwischen 0 und 50°C, bei normalem oder leicht erhöhtem Druck und gewöhnlich in Gegenwart eines gegenüber den Reaktionsteilnehmern inerten Lösungs- oder Verdünnungsmittels durchgeführt. Speziell geeignet sind unpolare und aprotische Lösungs- oder Verdünnungsmittel, z.B. Äther und ätherartige Verbindungen wie Diäthyläther, Di-isopropyläther, Dioxan und Tetrahydrofuran; aromatische Kohlenwasserstoffe, wie Benzol, Toluol und Xylole; Ketone wie Aceton, Methyläthylketon und Cyclohexanon, ferner Acetonitril, Dimethylsulfoxid und Dimethylformamid.

Die oben aufgezeigte Umsetzung erfolgt im allgemeinen in Gegenwart einer basischen Substanz, wobei man eine freie Verbindung der Formel I erhält. Arbeitet man ohne Basenzusatz, so gelangt man zu den entsprechenden Salzen, d.h. Hydrohalogeniden. Solche Hydrohalogenide können nach bekannten Methoden gegebenenfalls mit anderen Säuren in Salze der erwünschten Art übergeführt werden. Als basische Verbindungen sind anorganische Basen, wie KOH, NaOH, $NH_4OH$ oder Alkalimetallcarbonate, z.B. $NaHCO_3$, sowie organische Basen, wie Trialkylamine, z.B. Triäthylamin oder Äthyldiisopropylamin, Pyridin, Dialkylaniline usw., geeignet.

Die bei dem obigen Verfahren als Ausgangsstoffe einzusetzenden Verbindungen der Formeln II und III sind zum Teil bekannt; falls sie neu sind, können sie aus bekannten Vorläufern leicht nach üblichen Arbeitsweisen erhalten werden.

So kann man die Isothioharnstoffe der Formel II durch S-Alkylierung entsprechender Harnstoffe mit Alkyl-, Allyl- bzw. Propargylhalogeniden erhalten (vgl. US-Patentschrift Nr. 4,404,225, europäische Patentschrift Nr. 0,025,010 und deutsche Offenlegungsschrift Nr. 27 30 620).

In der US-Patentschrift 4,328,247 werden insektizide N-Phenoxyphenyl-N' -alkyl-isothioharnstoffe beschrieben. Es ist ferner aus der US-Patentschrift Nr. 4,357,351 bekannt, dass N-Phenyl-N'-acyl-isothioharnstoffe, deren N'-Stickstoff kein reaktives Wasserstoffatom aufweist, insektizide Eigenschaften mit verminderten phytotoxischen Nebeneffekten besitzen. Die erfindungsgemäss vorgeschlagenen Verbindungen sind demgegenüber neu und zeichnen sich durch hohe pestizide Wirksamkeit, speziell gegen Schadinsekten in Reis, aus.

Insbesondere eignen sich die Verbindungen der Formel I zur Bekämpfung von Insekten der Ordnungen: Lepidoptera, Coleoptera, Homoptera, Heteroptera, Diptera, Thysanoptera, Anoplura, Siphonaptera, Mallophaga, Thysanura, Isoptera, Psocoptera und Hymenoptera.

Die Verbindungen der Formel I sind weiterhin geeignet zur Bekämpfung von Vertretern der Ordnung Akarina der Familien: Ioxididae, Argasidae, Tetranychidae und Dermanyssidae. Die Verbindungen der Formel I können mit Erfolg vor allem zur Bekämpfung von phytopathogenen Milben, z.B. der Familien Tetranychidae und Phytoptipalpidae (Spinnmilben), Tarsonemidae (Fadenfussmilben) und Eriophydiae (Gallmilben), eingesetzt werden.

Neben ihrer Wirkung gegenüber Mücken und Fliegen, wie z.B. Aëdes aegypti und Musca domestica, sowie Aphiden können Verbindungen der Formel I auch zur Bekämpfung von pflanzenschädigenden Frassinsekten in Zier- und Nutzpflanzungen, insbesondere in Baumwollkulturen (z.B. gegen Spodoptera littoralis und Heliothis virescens) sowie in Getreide-, Obst- und Gemüsekulturen (z.B. gegen Laspeyresia pomonella, Leptinotarsa decemlineata und Epilachna varivestis) eingesetzt werden. Die Verbindungen der Formel I zeichnen sich auch durch eine gute Wirkung gegen larvale Insektenstadien und Nymphen, insbesondere von fressenden Schadinsekten, aus. Die erfindungsgemässen Verbindungen eignen sich weiterhin zur Bekämpfung von Bodeninsekten (z.B. Aulacophora femoralis, Chortophila brassicae, Diabrotica balteata, Pachnoda savignyi und

Scotia ypsilon).

Insbesondere können die Verbindungen der Formel I mit ausgezeichnetem Erfolg gegen pflanzenschädigende Zikaden, speziell in Reis-Kulturen, eingesetzt werden, wobei sie sowohl systemische als auch Kontakt-Wirkung zeigen.

Die Verbindungen der Formel I können ferner zur Bekämpfung von ektoparasitären Insekten und Akarinen an Haus- und Nutztieren verwendet werden, z.B. durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate. Mit besonderem Vorteil kann man Verbindungen der Formel I mit Diazinon, Dioxacarb, Heptenophos und Isoprocarb kombinieren, um zu wirksamen insektiziden Zubereitungen zu gelangen, die speziell zur Bekämpfung von Reiskulturen schädigenden Insekten geeignet sind.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, d.h. die den Wirkstoff der Formel I, bzw. Kombinationen dieser Wirkstoffe mit andern Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Äther und Ester, wie Äthanol, Äthylenglykol, Äthylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekornte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach der Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische, oberflächenaktive Verbindungen sein.

Als Seifen eignen sich die Alkali- Erdalkali- oder gegebenenfalls substituierten Ammoniumsaltze von höheren Fettsäuren ($C_{10}$-$C_{22}$), wie z.B. die Na- oder K-Salze der Öl- oder Stearinsäure, oder von natürlichen Fettsäuregemischen, die z.B. aus Kokosnuss- oder Tallöl gewonnen werden können. Weiterhin sind auch die Fettsäure-methyl-taurinsalze sowie modifizierte und nicht-modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Äthylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im aliphatischen Kohlenwasserstoffrest und 6 bis 18 Kohlenstoffatome im Alkylrest der Alkylphenole enthalten können. Weitere geeignete nichtionische Tenside

sind die wasserlöslichen 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylenpolyäthylenoxydaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan wie das Polyoxyäthylensorbitantrioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Diese Salze liegen vorzugsweise als Halogenide, Methylsulfate oder Äthylsulfate vor, z.B. das Stearyltrimethylammoniumchlorid oder das Benzyl-di-(2-chloräthyl)-äthylammoniumbromid.

Die in der Formulierungstechnik gebräuchlichen Tensiden sind u.a. in folgenden Publikationen beschrieben:
"Mc Cutcheon's Detergents and Emulsifiers Annual", MC Publishing Corp., Ridgewood, New Jersey, 1979;
Dr. Helmut Stache "Tensid Taschenbuch", Carl Hanser Verlag, München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelware eher konzentrierte Mittel bevorzugt werden, verwendet der Endverbraucher in der Regel verdünnte Mittel, die geringere Wirkstoffkonzentrationen aufweisen.

Die Mittel können auch weitere Zusätze, wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I oder Kombinationen dieser Wirkstoffe mit andern Insektiziden oder Akariziden (% = Gewichtsprozent)

| 1. Emulsionskonzentrate | a) | b) | c) |
|---|---|---|---|
| Wirkstoff resp. Wirkstoffkombination | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff resp. Wirkstoffkombination | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

3. Granulate

| | a) | b) |
|---|---|---|
| Wirkstoff resp. Wirkstoffkombination | 5 % | 10 % |
| Kaolin | 94 % | - |
| Hochdisperse Kieselsäure | 1 % | - |
| Attapulgit | - | 90 % |

Der Wirkstoff bzw. die Wirkstoffkombination wird in Methylenchlorid gelöst, auf den Träger aufgesprüht und das Lösungsmittel anschliessend im Vakuum abgedampft.

4. Stäubemittel

| | a) | b) |
|---|---|---|
| Wirkstoff resp. Wirkstoffkombination | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I resp. Kombinationen dieser Wirkstoffe mit andern Insektiziden oder Akariziden (% = Gewichtsprozent)

5. Spritzpulver

| | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination werden mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

| | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpqlyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

7. Stäubemittel

| | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

8. Extruder-Granulat

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

9. Umhüllungs-Granulat

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Beispiel 1**: Herstellung von N-(4-Phenoxy-2,6-diisopropylphenyl)-N'-tert.-butyl-N'-carbäthoxycarbonyl-S-methyl-isothioharnstoff

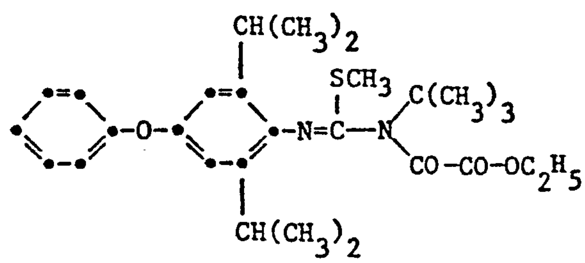

Es werden 9,9 g N-(4-Phenoxy-2,6-diisopropylphenyl)-N'-tert.-butyl-S-methylisothioharnstoff in 50 ml Methylenchlorid gelöst. Man tropft zu dieser Lösung 3,32 ml Triäthylamin und sodann bei 5-10°C 3,0 g Oxalsäureäthylesterchlorid. Das Reaktionsgemisch wird während drei Stunden bei Raumtemperatur gerührt und anschliessend mit Wasser gewaschen. Die abgetrennte organische Phase wird über $Na_2SO_4$ getrocknet und das Lösungsmittel abdestilliert. Das zurückbleibende Rohprodukt wird über eine Kieselgelkolonne mit einem Hexan:Äther-Gemisch (1:1) als Laufmittel chromatographiert. Man erhält die Titelverbindung als viskoses Öl, $n_D^{20}$: 1,5470 (Verbindung Nr. 1)

Analog der vorstehend beschriebenen Arbeitsweise werden die folgenden Verbindungen der Formel I hergestellt:

| Verb. Nr. | $R_1$ | $R_2$ | $R_3$ | $R_4$ | $R_5$ | $R_6$ | phys. Daten |
|---|---|---|---|---|---|---|---|
| 2 | $-CH(CH_3)_2$ | $-CH(CH_3)_2$ | H | $-CH_3$ | $-CH(CH_3)_2$ | $-CO-CO-OC_2H_5$ | $n_D^{20}$ = 1,5350 |
| 3 | $-CH(CH_3)_2$ | $-CH(CH_3)_2$ | 4-O- 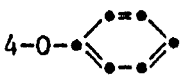 | $-CH_3$ | $-C(CH_3)_3$ | $-CO-CH_3$ | Smp. 91-93°C |
| 4 | $-C_2H_5$ | $-\underset{C_2H_5}{\underset{\mid}{C}H}-CH_3$ | H | $-CH_3$ | $-CH(CH_3)_2$ | $-CO-CO-OC_2H_5$ | $n_D^{20}$ = 1,5352 |
| 5 | $-C_2H_5$ | $-\underset{C_2H_5}{\underset{\mid}{C}H}-CH_3$ | H | $-CH_3$ | $-CH(CH_3)_2$ | $-CO-CH_3$ | $n_D^{20}$ = 1,5469 |
| 6 | $-C_2H_5$ | $-\underset{C_2H_5}{\underset{\mid}{C}H}-CH_3$ | H | $-CH_3$ | $-C(CH_3)_3$ | $-CO-CO-OC_2H_5$ | $n_D^{20}$ = 1,5372 |
| 7 | $-C_2H_5$ | $-\underset{C_2H_5}{\underset{\mid}{C}H}-CH_3$ | H | $-CH_3$ | $-C(CH_3)_3$ | $-CO-CH_3$ | $n_D^{20}$ = 1,5471 |
| 8 | $-C_2H_5$ | $-CH(CH_3)_2$ | H | $-CH_3$ | $-CH(CH_3)_2$ | $-CO-CH_3$ | $n_D^{20}$ = 1,5509 |
| 9 | $-C_2H_5$ | $-CH(CH_3)_2$ | H | $-CH_3$ | $-CH(CH_3)_2$ | $-CO-CO-OC_2H_5$ | $n_D^{20}$ = 1,5398 |
| 10 | $-CH_3$ | $-CH(CH_3)_2$ | H | $-CH_3$ | $-CH(CH_3)_2$ | $-CO-CO-OC_2H_5$ | $n_D^{20}$ = 1,5420 |
| 11 | $-CH_3$ | $-CH(CH_3)_2$ | H | $-CH_3$ | $-CH(CH_3)_2$ | $-CO-CH_3$ | $n_D^{20}$ = 1,5550 |
| 12 | $-CH(CH_3)_2$ | $-CH(CH_3)_2$ | H | $-CH_3$ | $-n-C_4H_9$ | $-CO-CH_3$ | $n_D^{20}$ = 1,5371 |
| 13 | $-CH_3$ | $-CH(CH_3)_2$ | H | $-CH_3$ | $-C(CH_3)_3$ | $-CO-CH_3$ | $n_D^{20}$ = 1,5478 |
| 14 | $-CH(CH_3)_2$ | $-CH(CH_3)_2$ | H | $-CH_3$ | $-n-C_4H_9$ | $-CO-CO-OC_2H_5$ | $n_D^{20}$ = 1,5295 |
| 15 | $-CH(CH_3)_2$ | $-CH(CH_3)_2$ | H | $-CH_3$ | $-CH_3$ | $-CO-CO-OC_2H_5$ | $n_D^{20}$ = 1,5426 |
| 16 | $-CH(CH_3)_2$ | $-CH(CH_3)_2$ | H | $-CH_3$ | $-CH_3$ | $-CO-CH_3$ | Smp. 84-86°C |
| 17 | $-C_2H_5$ | $-CH(CH_3)_2$ | H | $-CH_3$ | $-C(CH_3)_3$ | $-CO-CH_3$ | $n_D^{20}$ = 1,5502 |

| Verb. Nr. | $R_1$ | $R_2$ | $R_3$ | $R_4$ | $R_5$ | $R_6$ | phys. Daten |
|---|---|---|---|---|---|---|---|
| 18 | $-CH(CH_3)_2$ | $-CH(CH_3)_2$ | H | $-CH_3$ | $-C(CH_3)_3$ | $-CO-CO-OC_2H_5$ | $n_D^{20}$ = 1,5820 |
| 19 | $-CH(CH_3)_2$ | $-CH(CH_3)_2$ | H | $-CH_3$ | $-CH(CH_3)_2$ | $-CO-CO-OCH_3$ | Smp. ~ 70°C |
| 20 | $-CH(CH_3)_2$ | $-CH(CH_3)_2$ | H | $-CH_3$ | 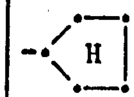 | $-CO-CO-OC_2H_5$ | $n_D^{21}$ = 1,5384 |
| 21 | $-CH(CH_3)_2$ | $-CH(CH_3)_2$ | H | $-CH_3$ | $-CH(CH_3)_2$ | $-CO-CH_3$ | $n_D^{21}$ = 1,5451 |
| 22 | $-C_2H_5$ | $-CH(C_2H_5)-CH_3$ | 4-O- 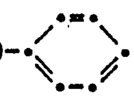 | $-CH_3$ | $-CH(CH_3)_2$ | $-CO-CH_3$ | $n_D^{21}$ = 1,5694 |
| 23 | $-CH(CH_3)_2$ | $-CH(CH_3)_2$ | H | $-CH_3$ | $-C(CH_3)_3$ | $-CO-CO-OCH_3$ | $n_D^{21}$ = 1,5327 |
| 24 | $-CH(CH_3)_2$ | $-CH(CH_3)_2$ | H | $-C_2H_5$ | $-CH(CH_3)_2$ | $-CO-CO-OC_2H_5$ | $n_D^{21}$ = 1,5208 |
| 25 | $-CH(CH_3)_2$ | $-CH(CH_3)_2$ | H | $-CH_3$ | $-CH(CH_3)_2$ | $-CO-CO-OC_4H_9$(i) | $n_D^{21}$ = 1,5248 |
| 26 | $-CH_3$ | $-CH_3$ | H | $-CH_3$ | $-CH(C_2H_5)-CH_3$ | $-CO-CO-OCH_3$ | $n_D^{21}$ = 1,5499 |
| 27 | $-CH_3$ | $-CH_3$ | H | $-CH_3$ | $-C(CH_3)_3$ | $-CO-CO-OCH_3$ | Smp. 62-70°C |
| 28 | $-CH(CH_3)_2$ | $-CH(CH_3)_2$ | 4-O- 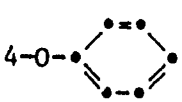 | $-CH_3$ | $-CH(CH_3)_2$ | $-CO-CO-OC_4H_9$(i) | $n_D^{23}$ = 1,5449 |
| 29 | $-CH(CH_3)_2$ | $-CH(CH_3)_2$ | 4-O- 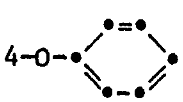 | $-CH_3$ | $-CH(CH_3)_2$ | $-CO-CH_2-CH(CH_3)_2$ | $n_D^{23}$ = 1,5498 |
| 30 | $-CH(CH_3)_2$ | $-CH(CH_3)_2$ | 4-Br | $-CH_3$ | $-CH(CH_3)_2$ | $-CO-CO-OC_2H_5$ | $n_D^{21}$ = 1,5510 |

Entsprechend dem vorstehend beschriebenen Verfahren sind auch folgende Verbindungen der Formel I herstellbar:

| Verb. Nr. | $R_1$ | $R_2$ | $R_3$ | $R_4$ | $R_5$ | $R_6$ |
|---|---|---|---|---|---|---|
| 31 | $-CH_3$ | $-CH_3$ | H | $-CH_3$ | $-C(CH_3)_3$ | $-CO-CO-OC_2H_5$ |
| 32 | $-CH_3$ | $-CH_3$ | 4-O- 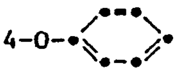 | $-CH_3$ | $-C(CH_3)_3$ | $-CO-CO-OC_2H_5$ |
| 33 | $-CH(CH_3)_2$ | $-CH(CH_3)_2$ | 4-O- 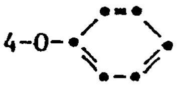 | $-CH_3$ | $-C(CH_3)_3$ | $-CO-CO-OCH_3$ |
| 34 | $-CH(CH_3)_2$ | $-CH(CH_3)_2$ | 4-O- 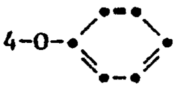 | $-CH_3$ | $-C(CH_3)_3$ | $-CO-CO-N(C_2H_5)_2$ |
| 35 | $-CH(CH_3)_2$ | $-CH(CH_3)_2$ | 4-O- 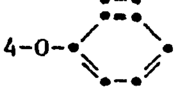 | $-CH_3$ | $-C(CH_3)_3$ | -CO- 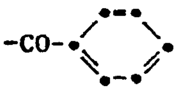 |
| 36 | $-CH(CH_3)_2$ | $-CH(CH_3)_2$ | 4-O- 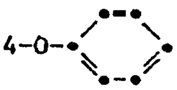 | $-CH_3$ | $-C(CH_3)_3$ | $-CO-OC_2H_5$ |
| 37 | $-CH(CH_3)_2$ | $-CH(CH_3)_2$ | 4-O- 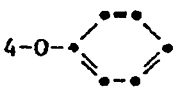 | $-CH_3$ | $-CH(CH_3)_2$ | $-SO_2-CH_3$ |
| 38 | $-CH(CH_3)_2$ | $-CH_3$ | 4-O- 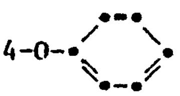 | $-CH_3$ | $-CH(CH_3)_2$ | $-CO-CO-N(CH_3)_2$ |
| 39 | $-C_2H_5$ | $-CH(CH_3)_2$ | H | $-C_2H_5$ | $-C(CH_3)_3$ | $-CO-CH_3$ |
| 40 | $-C_2H_5$ | $-CH(CH_3)_2$ | H | $-n-C_3H_7$ | $-C(CH_3)_3$ | $-CO-CH_3$ |
| 41 | $-C_2H_5$ | $-CH(CH_3)_2$ | H | $-CH_2-CH=CH_2$ | $-C(CH_3)_3$ | $-CO-CH_3$ |
| 42 | $-C_2H_5$ | $-CH(CH_3)_2$ | H | $-CH_2-C\equiv CH$ | $-C(CH_3)_3$ | $-CO-CH_3$ |
| 43 | $-CH(CH_3)_2$ | H | H | $-CH_3$ | $-C(CH_3)_3$ | $-CO-CO-OC_2H_5$ |
| 44 | $-CH(CH_3)_2$ | $-CH(CH_3)_2$ | 4-O- 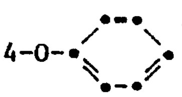 | $-CH_3$ | $-C(CH_3)_3$ | -CO- 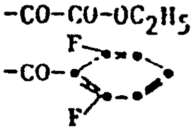 |

**Beispiel 2:** Wirkung gegen Laodelphax striatellus und Nilaparvate lugens (Nymphen)

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit jeweils 100 ml einer acetonischen Lösung enthaltend von 3 ppm bis 400 ppm ansteigende Konzentrationen des zu prüfenden Wirkstoffs besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein Glaszylinder gestülpt und dieser mit einem Gaze-Deckel abgedeckt. Die Nymphen werden bis zum Erreichen des folgenden Entwicklungsstadiums über 10 Tage an der behandelten Pflanze gehalten. Die Auswertung auf %-Mortalität erfolgt 1, 4 und 8 Tage nach der Behandlung.

Erfindungsgemässe Verbindungen zeigen in folgenden Konzentrationen gegen Nymphen von Nilaparvata lugens nach 8 Tagen 80-100 %ige Wirksamkeit (Mortalität):

| Verbindung Nr. | Wirkstoffkonzentration |
|---|---|
| 1 | 3 ppm |
| 2, 14 und 17 | 50 ppm |
| 18 | 100 ppm |
| 7 und 19 | 200 ppm |
| 15 | 400 ppm |

Mit den Verbindungen der Formel I gemäss Beispiel 1 kann auch gegen Nymphen von Laodelphax striatellus ebenfalls gute Wirksamkeit erzielt werden.

**Beispiel 3**: Wirkung gegen Laodelphax striatellus und Nilaparvata lugens (ovizid)

Der Test wird an wachsenden Pflanzen durchgeführt. Jeweils 4 Reispflanzen (Dicke des Stengels 8 mm, Höhe ca. 20 cm) werden in Töpfe (Durchmesser von 8 cm) eingepflanzt.

Die Pflanzen werden auf einem Drehteller mit jeweils 100 ml einer acetonischen Lösung enthaltend von 400 ppm Wirkstoff besprüht. Nach dem Antrocknen des Spritzbelages wird jede Pflanze mit 3 adulten Weibchen besiedelt. Um die Tiere am Entweichen zu hindern, wird über jede besiedelte Pflanze eim Glaszylinder gestülpt und dieser mit einem Gaze-Deckel abgedeckt. Die Weibchen bleiben 4 Tage zur Eiablage auf der behandelten Pflanze und werden dann entfernt.

Etwa 8 Tage nach Besiedlung schlüpfen die jungen Zikaden, und es erfolgt die Auszählung. Aus dem Vergleich der Anzahl geschlüpfter Larven auf den behandelten Pflanzen zu den auf unbehandelten Kontrollpflanzen geschlüpften Tieren wird die prozentuale Mortalität berechnet.

Verbindungen der Formel I gemäss Beispiel 1 zeigen in obigem Test gute ovizide Wirkung.

**Beispiel 4**: Insektizide Kontaktwirkung gegen Aphis craccivora

In Töpfen angezogene Pflanzen (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 50 und 200 ppm der zu prüfenden Verbindung bis zur Tropfnässe besprüht. Man verwendet pro Test-Verbindung und pro Konzentration zwei Pflanzen. Die Auswertung der erzielten Abtötungsrate erfolgt nach weiteren 24 Stunden.

Eine 80-100 %ige Abtötung wird mit Verbindung Nr. 6 bei 50 ppm und mit Verbindung Nr. 17 bei 200 ppm erzielt.

**Beispiel 5**: Wirkung gegen Lucilia sericata

Zu 9 ml eines Zuchtmediums werden bei 50°C 1 ml einer 0,5 % Aktivsubstanz enthaltenden wässrigen Lösung gegeben. Nach einer Stunde werden ca. 30 frisch geschlüpfte Lucilia sericata-Larven zum Zuchtmedium gegeben, und nach 48 und 96 Std. wird die insektizide Wirkung durch Ermittlung der Abtötungsrate festgestellt.

Verbindungen der Formel I gemäss Beispiel 1 zeigen in diesem Test gute Wirkung gegen Lucilia sericata.

**Beispiel 6**: Wirkung gegen Aëdes aegypti:

Auf die Oberfläche von 150 ml Wasser, das sich in einem Behälter befindet, wird so viel einer 0,1 %igen acetonischen Lösung des Wirkstoffes pipettiert, dass eine Konzentration von 800 ppm erhalten wird. Nach Verdunsten des Acetons wird der Behälter mit 30 bis 40 2-tägigen Aëdes-Larven beschickt. Nach 1, 2 und 5

Tagen wird die Mortalität geprüft.

Verbindungen der Formel I gemäss Beispiel 1 zeigen in diesem Test gute Wirkung gegen Aëdes aegypti.

**Beispiel 7:** Insektizide Frassgift-Wirkung

Ca. 25 cm hohe eingetopfte Baumwollpflanzen werden mit wässrigen Wirkstoffemulsionen besprüht, die den Wirkstoff in Konzentrationen von 50 bzw. 400 ppm enthalten.

Nach dem Antrocknen des Sprühbelages werden die Baumwollpflanzeen mit Spodoptera littoralis- bzw. Heliothis virescens-Larven im dritten larvalen Stadium besiedelt. Der Versuch wird bei 24° C und 60 % relativer Luftfeuchtigkeit durchgeführt. Nach 120 Stunden wird die %-Mortalität der Test-Insekten bestimmt.

Die Verbindungen Nr. 1 und 3 zeigen 80-100 % Wirkung (Mortalität) gegen Spodoptera-Larven bei 50 ppm und gegen Heliothis-Larven bei 400 ppm.

**Beispiel 8:** Wirkung gegen Spinnmilben

Phaseolus vulgaris-Pflanzen werden 12 Stunden vor dem Test auf akarizide Wirkung mit einem infestierten Blattstück aus einer Massenzucht von Tetranychus urticae belegt. Die übergelaufenen beweglichen Stadien werden aus einem Chromatographiezerstäuber mit den emulgierten Testpräparaten (Wirkstoffkonzentration 400 ppm) derart besprüht, dass kein Ablaufen der Spritzbrühe eintritt. Nach zwei und 7 Tagen werden Larven, Adulte und Eier unter dem Binokular auf lebende und tote Individuen ausgewertet und das Ergebnis in Prozenten ausgedrückt. Während der "Haltezeit" stehen die behandelten Pflanzen in Gewächshauskabinen bei 25° C.

Verbindungen der Formel I gemäss Beispiel 1 wirken im obigen Test gegen Adulte, Larven und Eier von Tetranychus urticae.

**Beispiel 9:** Wirkung gegen Zecken

a) Rhipicephalus bursa

Je 5 adulte Zecken bzw. 50 Zeckenlarven werden in ein Glassröhrchen gezählt und für 1 bis 2 Minuten in 2 ml einer wässrigen Emulsion enthaltend 800 ppm der Testsubstanz getaucht. Das Röhrchen wird dann mit einem genormten Wattebausch verschlossen und auf den Kopf gestellt, damit die Wirkstoffemulsion von der Watte aufgenommen werden konnte.

Die Auswertung erfolgt bei den Adulten nach 2 Wochen und bei den Larven nach 2 Tagen. Für jeden Versuch laufen 2 Wiederholungen.

b) Boophilus microplus

Mit einer analogen Wirkstoffkonzentration wie unter a) beschrieben werden mit je 20 sensiblen resp. OPresisten Larven Tests durchgeführt (Die Resistenz bezieht sich auf die Verträglichkeit gegen Diazinon).

Verbindungen der Formel I gemäss Beispiel 1 zeigen gute Wirkung in den obigen Tests a) und b).

**Beispiel 10:** Ovizide Wirkung auf Spodoptera littoralis

Auf Filterpapier abgelegte Eier von Spodoptera littoralis werden aus dem Papier ausgeschnitten und in eine 0,05 Gew.-%ige Lösung des Wirkstoffes in einem Aceton-Wasser-Gemisch (1:1) getaucht. Die so behandelten Eiablagen werden dann aus diesem Gemisch herausgenommen und bei 28° C und 60 % relativer Feuchtigkeit in Kunststoffschalen deponiert.

Nach 5 Tagen wird die Schlupfrate, d.h. die Anzahl Larven, die sich aus den behandelten Eiern entwickelt haben, bestimmt.

Verbindungen der Formel I gemäss Beispiel 1 zeigen gute Wirkung im obigen Test.

**Beispiel 11:** Ovizide Wirkung auf Heliothis virescens und Leptinotarsa decemlineata

Entsprechende Mengenanteile einer benetzbaren pulverförmigen Formulierung, enthaltend 25 Gew.-% des zu prüfenden Wirkstoffes, werden mit jeweils soviel Wasser vermischt, dass sich wässrige Emulsionen mit einer Wirkstoffkonzentration von 800 ppm ergeben.

In diese wirkstoffhaltigen Emulsionen werden eintägige Eigelege von Heliothis auf Cellophan ® bzw. Eigelege von Leptinotarsa auf Kartoffelblättern während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgestellt (Auswertung auf 80-100%ige Abtötung der Eier).

Verbindungen der Formel I gemäss Beispiel 1 zeigen gute Wirkung in obigem Test.

**Beispiel 12:** Wirkung gegen Bodeninsekten (Diabrotica balteata)

Es werden 350 ml Erde (bestehend aus 95 Vol.-% Sand und 5 Vol.-% Torf) mit jeweils 150 ml von wässrigen Emulsionszubereitungen vermischt, die den zu prüfenden Wirkstoff in steigenden Konzentrationen von 3 ppm bis 200 ppm enthalten. Dann werden Plastikbecher, die einen oberen Durchmesser von ca. 10 cm haben, mit der so behandelten Erde teilweise gefüllt. Pro Becher werden zehn Larven von Diabrotica balteata im dritten Larvalstadium eingesetzt, vier Maiskeimlinge eingepflanzt und die Becher mit Erde aufgefüllt. Die gefüllten Becher werden mit Plastikfolie abgedeckt und bei einer Temperatur von etwa 22°C gehalten. Zehn Tage nach dem Ansatz wird die in den Bechern enthaltene Erde abgesiebt und die zurückbleibenden Larven hinsichtlich ihrer Mortalität geprüft.

Erfindungsgemässe Verbindungen zeigen in diesem Test bei den nachstehend aufgeführten Konzentrationen eine Wirkung von 80-100 % (Mortalität):

| Verbindung Nr. | Wirkstoffkonzentration |
|---|---|
| 1 | 3 ppm |
| 6, 18 und 19 | 12,5 ppm |
| 2, 4, 9, 10, 13, 14 und 17 | 50 ppm |
| 7 | 100 ppm |
| 8 und 15 | 200 ppm |

**Patentansprüche** für die Vertragsstaaten: BE, CH, DE, FR, GB, IT, LI, NL

1. Verbindung der Formel I

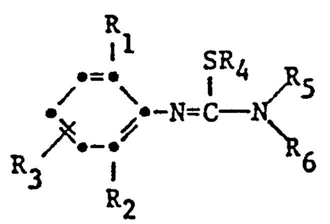

(I),

worin

$R_1$ $C_1$-$C_5$-Alkyl;

$R_2$ Wasserstoff oder $C_1$-$C_5$-Alkyl;

$R_3$ Wasserstoff, Halogen, $C_1$-$C_5$-Alkyl, Phenoxy oder durch 1 oder Halogenatome, $C_1$-$C_5$-Alkyl- oder Trifluormethylreste substituiertes Phenoxy;

$R_4$ $C_1$-$C_5$-Alkyl, Allyl oder Propargyl;

$R_5$ $C_1$-$C_5$-Alkyl, $C_3$-$C_6$-Cycloalkyl, Allyl oder Propargyl; und

$R_6$ einen der Reste

$-CO-R_7$, $-CO-CO-R_8$, $-SO_2-R_7$ oder $-CO-OR_7$, bedeuten, wobei

$R_7$ $C_1$-$C_5$-Alkyl, Phenyl oder durch 1 oder 2 Halogenatome oder Methylreste substituiertes Phenyl, und

$R_8$ $C_1$-$C_{10}$-Alkoxy oder Dialkylamino mit $C_1$-$C_5$-Alkylgruppen bedeuten.

2. Verbindung gemäss Anspruch 1 der Formel I

worin
$R_1$ $C_1$-$C_4$-Alkyl;
$R_2$ Wasserstoff oder $C_1$-$C_5$-Alkyl;
$R_3$ Wasserstoff, Fluor, Chlor, $C_1$-$C_3$-Alkyl, Phenoxy oder durch 1 oder 2 Fluor- oder Chloratome, Methyl-, Äthyl-oder Trifluoremethylreste substituiertes Phenoxy;
$R_4$ $C_1$-$C_3$-Alkyl, Allyl oder Propargyl;
$R_5$ $C_2$-$C_5$-Alkyl, $C_3$-$C_6$-Cycloalkyl, Allyl oder Propargyl; und
$R_6$ einen der Reste
-CO-$R_7$, -CO-CO-$R_8$, -$SO_2$-$R_7$ oder -CO-O$R_7$,
bedeuten, wobei
$R_7$ $C_1$-$C_4$-Alkyl, Phenyl oder durch 1 oder 2 Fluor- oder Chloratome oder Methylreste substituiertes Phenyl, und
$R_8$ $C_1$-$C_4$-Alkoxy oder Dialkylamino mit $C_1$-$C_3$-Alkylgruppen
bedeuten.

3. Verbindung gemäss Anspruch 2 der Formel I,
worin
$R_1$ $C_1$-$C_4$-Alkyl;
$R_2$ $C_1$-$C_5$-Alkyl;
$R_3$ Wasserstoff, Fluor, Chlor, Methyl, Phenoxy, durch ein Fluor- oder Chloratom, einen Methyl- oder Trifluormethylrest substituiertes Phenoxy;
$R_4$ $C_1$-$C_3$-Alkyl, Allyl oder Propargyl;
$R_5$ $C_2$-$C_5$-Alkyl, Cyclopropyl, Cyclohexyl, Allyl oder Propargyl; und
$R_6$ einen der Reste
-CO-$R_7$ oder -CO-CO-$R_8$ bedeuten, wobei
$R_7$ Methyl, Äthyl, Phenyl oder durch ein oder zwei Fluor- oder Chloratome substituiertes Phenyl; und
$R_8$ Methoxy, Aethoxy oder Dialkylamino mit Methyl- oder Aethylgruppen
bedeuten.

4. Verbindung gemäss Anspruch 3, der Formel I, worin
$R_1$ $C_1$-$C_3$-Alkyl;
$R_2$ $C_1$-$C_5$-Alkyl;
$R_3$ Wasserstoff oder 4-Phenoxy;
$R_4$ $C_1$-$C_3$-Alkyl, Allyl oder Propargyl;
$R_5$ $C_2$-$C_5$-Alkyl;
$R_6$ einen der Rest
-CO-$R_7$ oder -CO-CO-$R_8$ bedeuten, wobei
$R_7$ Methyl, Äthyl, Phenyl oder Difluorphenyl und
$R_8$ Methoxy, Äthoxy, Dimethylamino oder Diathylamino bedeuten.

5. Verbindung gemäss Anspruch 4, dadurch gekennzeichnet, dass
$R_1$ $C_1$-$C_3$-Alkyl;
$R_2$ $C_2$-$C_4$-Alkyl;
$R_3$ Wasserstoff oder 4-Phenoxy;
$R_4$ Methyl;
$R_5$ $C_1$-$C_4$-Alkyl; und
$R_6$ einen der Reste -CO-$CH_3$ oder -CO-CO-O$C_2H_5$ bedeuten.

6. Verbindung gemäss Anspruch 5 der Formel

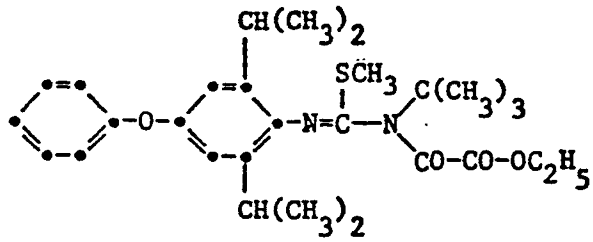

7. Verbindung gemäss Anspruch 5 der Formel

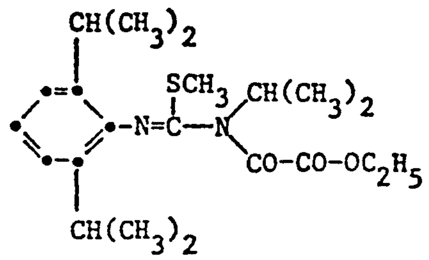

8. Verbindung gemäss Anspruch 5 der Formel

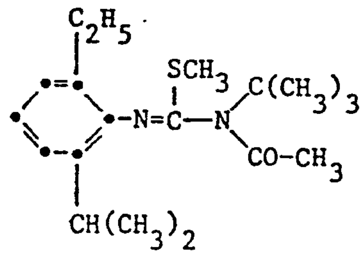

9. Verfahren zur Herstellung einer Verbindung der Formel I gemäss Anspruch 1 bis 8 dadurch gekennzeichnet, dass man eine Verbindung der Formel II

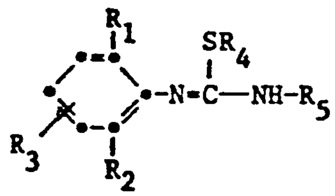

(II)

mit einer Verbindung der Formel III

X - $R_6$ (III)

umsetzt wobei in den Formeln II und III $R_1$ bis $R_6$ die in den Anspruchen 1 bis 5 angegebenen Bedeutungen haben und X für Halogen, vorzugsweise Chlor, steht.

10. Mittel zur Bekämpfung von pflanzenschädigenden Insekten und Vertretern der Ordnung Akarina, welches als aktive Komponente eine Verbindung gemäss den Ansprüchen 1 bis 8 zusammen mit geeignetem Träger- und/oder anderen Zuschlagstoffen enthält.

11. Verwendung einer Verbindung gemäss den Ansprüchen 1 bis 8 zur Bekämpfung pflanzenschädigender Insekten und Vertreterm der Ordnung Akarina.

12. Verwendung gemäss Anspruch 11 zur Bekämpfung von pflanzenschädigenden Zikaden und Bodeninsekten.

13. Verwendung gemäss Anspruch 12 zur Bekämpfung von Zikaden in Reiskulturen.

14. Verfahren zur Bekämpfung von Insektem und Vertretern der Ordnung Akarina, dadurch gekennzeichnet, dass man die Schädlinge bzw. deren verschiedene Entwicklungsstadien oder deren Aufenthaltsort mit einer pestizid wirksamen Menge einer Verbindung der Formel I gemäss einem der Ansprüche 1 bis 8 oder mit einem Mittel enthaltend neben Zusatz- und Trägerstoffen eine pestizid wirksame Menge dieser Verbindung, in Kontakt bringt oder behandelt.

**Patentansprüche** für den Vertragsstaat: AT

1. Verfahren zur Herstellung einer Verbindung der Formel I

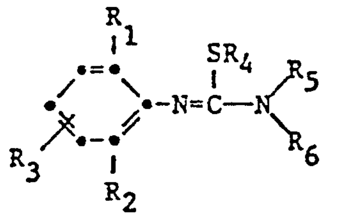

(I),

worin
$R_1$ $C_1$-$C_5$-Alkyl;
$R_2$ Wasserstoff oder $C_1$-$C_5$-Alkyl;
$R_3$ Wasserstoff, Halogen, $C_1$-$C_5$-Alkyl, Phenoxy oder durch 1 oder 2 Halogenatome, $C_1$-$C_5$-Alkyl- oder Trifluormethylreste substituiertes Phenoxy;
$R_4$ $C_1$-$C_5$-Alkyl, Allyl oder Propargyl;
$R_5$ $C_1$-$C_5$-Alkyl, $C_3$-$C_6$-Cycloalkyl, Allyl oder Propargyl; und
$R_6$ einen der Reste
-CO-$R_7$, -CO-CO-$R_8$, -$SO_2$-$R_7$ oder -CO-O$R_7$,
bedeuten, wobei
$R_7$ $C_1$-$C_5$-Alkyl, Phenyl oder durch 1 oder 2 Halogenatome oder Methylreste substituiertes Phenyl, und
$R_8$ $C_1$-$C_{10}$-Alkoxy oder Dialkylamino mit $C_1$-$C_5$-Alkylgruppen bedeuten, dadurch gekennzeichnet, dass man eine Verbindung der Formel II

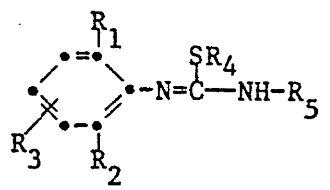

(II)

mit einer Verbindung der Formel III
X - $R_6$ (III)
umsetzt, wobei in den Formeln II und III $R_1$ bis $R_6$ die oben angegebenen Bedeutungen haben und X für Halogen, vorzugsweise Chlor steht.

2. Verfahren gemäss Anspruch 1 zur Herstellung einer Verbindung der Formel I
worin
$R_1$ $C_1$-$C_4$-Alkyl;
$R_2$ Wasserstoff oder $C_1$-$C_5$-Alkyl;
$R_3$ Wasserstoff, Fluor, Chlor, $C_1$-$C_3$-Alkyl, Phenoxy oder durch 1 oder 2 Fluor- oder Chloratome, Methyl-, Äthyl oder Trifluoremethylreste substituiertes Phenoxy;
$R_4$ $C_1$-$C_3$-Alkyl, Allyl oder Propargyl;
$R_5$ $C_2$-$C_5$-Alkyl, $C_3$-$C_6$-Cycloalkyl, Allyl oder Propargyl; und
$R_6$ einen der Reste
-CO-$R_7$, -CO-CO-$R_8$, -$SO_2$-$R_7$ oder -CO-O$R_7$,
bedeuten, wobei
$R_7$ $C_1$-$C_4$-Alkyl, Phenyl oder durch 1 oder 2 Fluor- oder Chloratome oder Methylreste substituiertes Phenyl, und
$R_8$ $C_1$-$C_4$-Alkoxy, oder Dialkylamino mit $C_1$-$C_3$ -Alkylgruppen
bedeuten.

3. Verfahren gemäss Anspruch 2 zur Herstellung einer Verbindung der Formel I,
worin
$R_1$ $C_1$-$C_4$-Alkyl;
$R_2$ $C_1$-$C_5$-Alkyl;
$R_3$ Wasserstoff, Fluor, Chlor, Methyl, Phenoxy, durch ein Fluor- oder Chloratom, einen Methyl- oder Trifluormethylrest substituiertes Phenoxy;
$R_4$ $C_1$-$C_3$-Alkyl, Allyl oder Propargyl;
$R_5$ $C_2$-$C_5$-Alkyl, Cyclopropyl, Cyclohexyl, Allyl oder Propargyl; und
$R_6$ einen der Reste
-CO-$R_7$ oder -CO-CO-$R_8$ bedeuten, wobei
$R_7$ Methyl, Äthyl, Phenyl oder durch ein oder zwei Fluor- oder Chloratome substituiertes Phenyl; und
$R_8$ Methoxy, Äthoxy oder Dialkylamino mit Methyl- oder Äthylgruppen
bedeuten.

4. Verfahren gemäss Anspruch 3 zur Herstellung einer Verbindung der Formel I, worin
$R_1$ $C_1$-$C_3$-Alkyl;
$R_2$ $C_1$-$C_5$-Alkyl;
$R_3$ Waßserstoff oder 4-Phenoxy;
$R_4$ $C_1$-$C_3$-Alkyl, Allyl oder Propargyl;
$R_5$ $C_2$-$C_5$-Alkyl;
$R_6$ einen der Reste
-CO-$R_7$ oder -CO-CO-$R_8$ bedeuten wobei
$R_7$ Methyl, Äthyl, Phenyl oder Difluorphenyl und
$R_8$ Methoxy, Äthoxy, Dimethylamino oder Diäthylamino bedeuten.
5. Verfahren gemäss Anspruch 4 zur Herstellung einer Verbindung der Formel I, worin
$R_1$ $C_1$-$C_3$-Alkyl;
$R_2$ $C_2$-$C_4$-Alkyl;
$R_3$ Wasserstoff oder 4-Phenoxy;
$R_4$ Methyl;
$R_5$ $C_1$-$C_4$-Alkyl; und
$R_6$ einen der Reste -CO-$CH_3$ oder -CO-CO-$OC_2H_5$
bedeuten.

6. Verfahren gemäss Anspruch 5 zur Herstellung der Verbindung der Formel

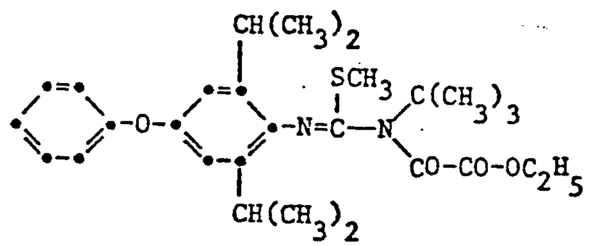

7. Verfahren gemäss Anspruch 5 zur Herstellung, der Verbindung der Formel

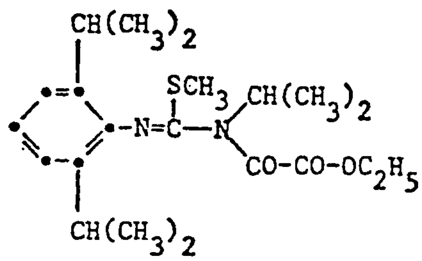

8. Verfahren gemäss Anspruch 5 zur Herstellung der Verbindung der Formel

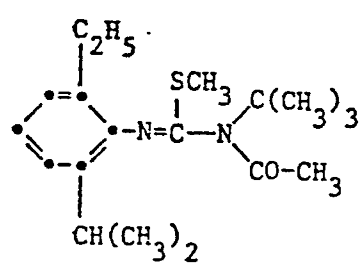

9. Mittel zur Bekämpfung von pflanzenschädigenden Insekten und Vertreterm der Ordnung Akarina, welches als aktive Komponente eine Verbindung gemäss den Ansprüchem 1 bis 8 zusammen mit geeignetem Träger- und/oder anderen Zuschlagstoffen enthält.

10. Verwendung einer Verbindung gemäss den Anspruchen 1 bis 8 zur Bekämpfung pflanzenschädigender Insekten und Vertreterm der Ordnung Akarina.

11. Verwendung gemäss Anspruch 10 zur Bekämpfung von pflanzenschädigenden Zikaden und Bodeninsekten.

12. Verwendung gemäss Anspruch 11 zur Bekämpfung von Zikaden in Reiskulturen.

13. Verfahren aur Bekämpfung von Insekten und Vertretern der Ordnung Akarina, dadurch gekennzeichnet, dass man die Schädlinge bzw. deren verschiedene Entwicklungsstadien oder deren Aufenthaltsort mit einer pestizid wirksamen Menge einer Verbindung der Formel I gemäss einem der Ansprüche 1 bis 8 oder mit einem Mittel enthaltend neben Zusatz- und Trägerstoffen eine pestizid wirksame Menge dieser Verbindung in Kontakt bringt oder behandelt.

**Claims for the Contracting States**: BE, CH, DE, FR, GB, IT, LI, NL

1. A compound of formula I

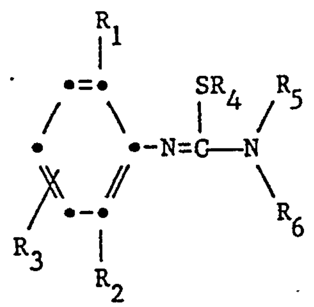

(I),

wherein
$R_1$ is $C_1$-$C_5$alkyl,
$R_2$ is hydrogen or $C_1$-$C_5$alkyl,
$R_3$ is hydrogen, halogen, $C_1$-$C_5$alkyl, phenoxy or phenoxy which is substituted by 1 or 2 identical or different members selected from the group consisting of halogen atoms, $C_1$-$C_5$alkyl or trifluoromethyl radicals,
$R_4$ is $C_1$-$C_5$alkyl, allyl or propargyl,
$R_5$ is $C_1$-$C_5$alkyl, $C_3$-$C_6$cycloalkyl, allyl or propargyl, and
$R_6$ is a radical selected from -CO-$R_7$, -CO-CO-$R_8$, -$SO_2$-$R_7$ or -CO-O$R_7$, wherein
$R_7$ is $C_1$-$C_5$alkyl, phenyl or phenyl which is substituted by 1 or 2 halogen atoms or methyl groups, and
$R_8$ is $C_1$-$C_{10}$alkoxy or di($C_1$-$C_5$)alkylamino.

2. A compound according to claim 1 of formula I, wherein
$R_1$ is $C_1$-$C_4$alkyl,
$R_2$ is hydrogen or $C_1$-$C_5$alkyl,
$R_3$ is hydrogen, fluorine, chlorine, $C_1$-$C_3$alkyl, phenoxy or phenoxy which is substituted by 1 or 2 identical or different members selected from the group consisting of fluorine or chlorine atoms, methyl, ethyl or trifluoromethyl groups,
$R_4$ is $C_1$-$C_3$alkyl, allyl or propargyl,
$R_5$ is $C_2$-$C_5$alkyl, $C_3$-$C_6$cycloalkyl, allyl or propargyl, and
$R_6$ is a radical selected from -CO-$R_7$, -CO-CO-$R_8$, -$SO_2$-$R_7$ or -CO-O$R_7$, wherein
$R_7$ is $C_1$-$C_4$alkyl, phenyl or phenyl which is substituted by 1 or identical or different members selected from the group consisting of fluorine or chlorine atoms or methyl groups, and
$R_8$ is $C_1$-$C_4$alkoxy or di($C_1$-$C_3$)alkylamino.

3. A compound according to claim 2, of the formula I, wherein
$R_1$ is $C_1$-$C_4$alkyl,
$R_2$ is $C_1$-$C_5$alkyl,
$R_3$ is hydrogen, fluorine, chlorine, methyl, phenoxy or phenoxy which is substituted by a member selected from the group consisting of fluorine, chlorine, methyl or trifluoromethyl,
$R_4$ is $C_1$-$C_3$alkyl, allyl or propargyl,
$R_5$ is $C_2$-$C_5$alkyl, cyclopropyl, cyclohexyl, allyl or propargyl, and
$R_6$ is a radical selected from -CO-$R_7$ or -CO-CO-$R_8$, wherein
$R_7$ is methyl, ethyl, phenyl or phenyl which is substituted by 1 or 2 fluorine or chlorine atoms, and
$R_8$ is methoxy, ethoxy, or dialkylamino in which alkyl is methyl or ethyl.

4. A compound according to claim 3, of the formula I, wherein
$R_1$ is $C_1$-$C_3$alkyl,
$R_2$ is $C_2$-$C_5$alkyl,
$R_3$ is hydrogen or 4-phenoxy,
$R_4$ is $C_1$-$C_3$alkyl, allyl or propargyl,
$R_5$ is $C_2$-$C_5$alkyl,
$R_6$ is a radical selected from -CO-$R_7$ or -CO-CO-$R_8$, wherein
$R_7$ is methyl, ethyl, phenyl or difluorophenyl, and
$R_8$ is methoxy, ethoxy, dimethylamino or diethylamino.

5. A compound according to claim 4, wherein
$R_1$ is $C_1$-$C_3$ alkyl
$R_2$ is $C_2$-$C_4$alkyl,
$R_3$ is hydrogen or 4-phenoxy,
$R_4$ is methyl,
$R_5$ is $C_1$-$C_4$alkyl, and
$R_6$ is a $-CO-CH_3$ or $-CO-CO-OC_2H_5$ radical.

6. A compound according to claim 5, of the formula

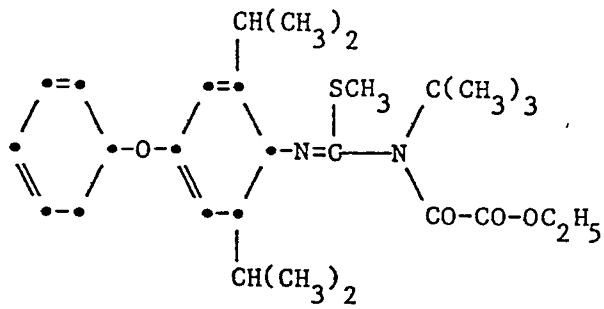

7. A compound according to claim 5, of the formula

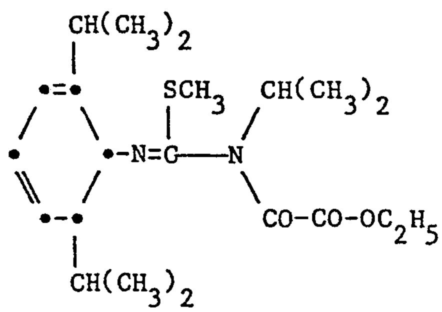

8. A compound according to claim 5, of the formula

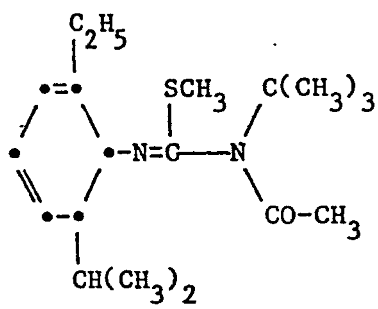

9. A process for the preparation of a compound of the formula I according to any one of claims 1 to 8, which comprises reacting a compound of formula II

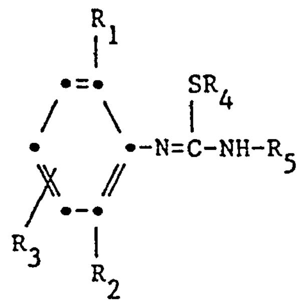

(II)

with a compound of formula III

$X - R_6$ (III)

in which formulae above $R_1$ to $R_6$ are as defined in claims 1 to 5 and X is halogen, preferably chlorine.

10. A composition for controlling plant destructive insects and representatives of the order Acarina, which composition contains a compound as claimed in any one of claims 1 to 8, together with suitable carriers and/or other adjuvants.

11. A method of controlling plant destructive insects and representatives of the order Acarina at a locus, which comprises applying to said locus a pesticidally effective amount of a compound as claimed in any one of claims 1 to 8.

12. A method according to claim 11, wherein the pests to be controlled are plant destructive cicadas and soil insects.

13. A method according to claim 12, wherein the cicadas to be controlled occur in rice crops.

14. A method of controlling insects and representatives of the order Acarina, which comprises contacting these pests, their various development stages or the locus thereof with a pesticidally effective amount of a compound of formula I according to any one of claims 1 to 8 or with a composition which contains a pesticidally effective amount of such a compound together with adjuvants and carriers.

**Claims for the Contracting State**: AT

1. A process for the preparation of a compound of formula I

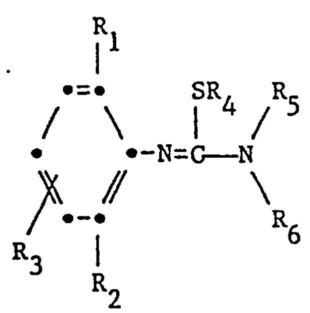

(I),

wherein

$R_1$ is $C_1$-$C_5$alkyl,

$R_2$ is hydrogen or $C_1$-$C_5$alkyl,

$R_3$ is hydrogen halogen $C_1$-$C_5$alkyl, phenoxy or phenoxy which is substituted by 1 or 2 identical or different members selected from the group consisting of halogen atoms, $C_1$-$C_5$alkyl or trifluoromethyl radicals,

$R_4$ is $C_1$-$C_5$alkyl, allyl or propargyl,

$R_5$ is $C_1$-$C_5$alkyl, $C_3$-$C_6$cycloalkyl, allyl or propargyl, and

$R_6$ is a radical selected from -CO-$R_7$, -CO-CO-$R_8$, -$SO_2$-$R_7$ or -CO-O$R_7$, wherein

$R_7$ is $C_1$-$C_5$alkyl, phenyl or phenyl which is substituted by 1 or 2 halogen atoms or methyl groups, and

$R_8$ is $C_1$-$C_{10}$alkoxy or di($C_1$-$C_5$)alkylamino,

which comprises reacting a compound of formula II

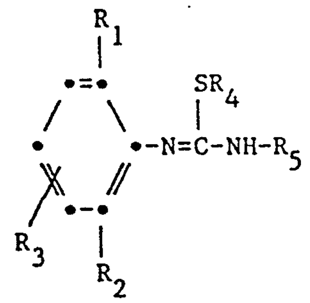

(II)

with a compound of formula III

X - $R_6$ (III)

in which formulae above $R_1$ to $R_6$ are as defined above and X is halogen, preferably chlorine.

2. A process according to claim 1 for the preparation of a compound of formula I, wherein
$R_1$ is $C_1$-$C_4$alkyl,
$R_2$ is hydrogen or $C_1$-$C_5$alkyl,
$R_3$ is hydrogen, fluorine, chlorine, $C_1$-$C_3$alkyl, phenoxy or phenoxy which is substituted by 1 or 2 identical or different members selected from the group consisting of fluorine or chlorine atoms, methyl, ethyl or trifluoromethyl groups,
$R_4$ is $C_1$-$C_3$alkyl, allyl or propargyl,
$R_5$ is $C_2$-$C_5$alkyl, $C_3$-$C_6$cycloalkyl, allyl or propargyl, and
$R_6$ is a radical selected from -CO-$R_7$, -CO-CO-$R_8$, -$SO_2$-$R_7$ or -CO-O$R_7$, wherein
$R_7$ is $C_1$-$C_4$alkyl, phenyl or phenyl which is substituted by 1 or 2 identical or different members selected from the group consisting of fluorine or chlorine atoms or methyl groups, and
$R_8$ is $C_1$-$C_4$alkoxy or di($C_1$-$C_3$)alkylamino.

3. A process according to claim 2 for the preparation of a compound of formula I, wherein
$R_1$ is $C_1$-$C_4$alkyl,
$R_2$ is $C_1$-$C_5$alkyl,
$R_3$ is hydrogen, fluorine, chlorine, methyl, phenoxy or phenoxy which is substituted by a member selected from the group consisting of fluorine, chlorine, methyl or trifluoromethyl,
$R_4$ is $C_1$-$C_3$alkyl, allyl or propargyl,
$R_5$ is $C_2$-$C_5$alkyl, cyclopropyl, cyclohexyl, allyl or propargyl, and
$R_6$ is a radical selected from -CO-$R_7$ or -CO-CO-$R_8$, wherein
$R_7$ is methyl, ethyl, phenyl or phenyl which is substituted by 1 or 2 fluorine or chlorine atoms, and
$R_8$ is methoxy, ethoxy, or dialkylamino in which alkyl is methyl or ethyl.

4. A process according to claim 3 for the preparation of a compound of formula I, wherein
$R_1$ is $C_1$-$C_3$alkyl,
$R_2$ is $C_2$-$C_5$alkyl,
$R_3$ is hydrogen or 4-phenoxy,
$R_4$ is $C_1$-$C_3$alkyl, allyl or propargyl,
$R_5$ is $C_2$-$C_5$alkyl,
$R_6$ is a radical selected from -CO-$R_7$ or -CO-CO-$R_8$, wherein
$R_7$ is methyl, ethyl, phenyl or difluorophenyl, and
$R_8$ is methoxy, ethoxy, dimethylamino or diethylamino.

5. A process according to claim 4 for the preparation of a compound of formula I, wherein
$R_1$ is $C_1$-$C_3$ alkyl,
$R_2$ is $C_2$-$C_4$alkyl
$R_3$ is hydrogen or 4-phenoxy,
$R_4$ is methyl,
$R_5$ is $C_1$-$C_4$ alkyl and
$R_6$ is a -CO-$CH_3$ or -CO-CO-$OC_2H_5$ radical.

6. A process according to claim 5 for the preparation of the compound of formula

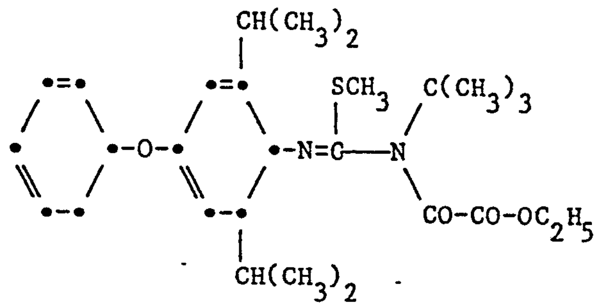

7. A process according to claim 5 for the preparation of the compound of formula

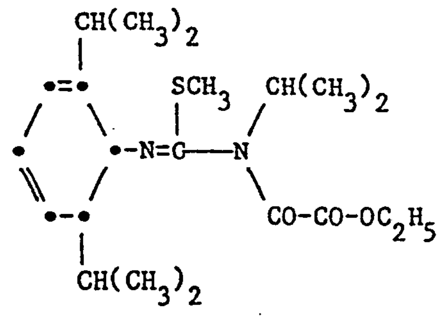

8. A process according to claim 5 for the preparation of the compound of formula

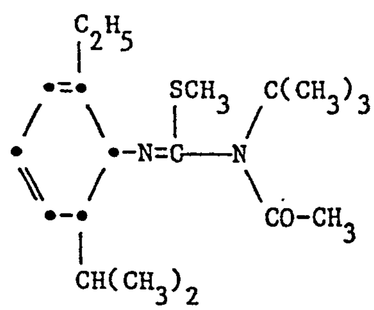

9. A composition for controlling plant destructive insects and representatives of the order Acarina, which composition contains a compound as claimed in any one of claims 1 to 8, together with suitable carriers and/or other adjuvants.

10. A method of controlling plant destructive insects and representatives of the order Acarina at a locus, which comprises applying to said locus a pesticidally effective amount of a compound as claimed in any one of claims 1 to 8.

11. A method according to claim 10, wherein the pests to be controlled are plant destructive cicadas and soil insects.

12. A method according to claim 11, wherein the cicadas to be controlled occur in rice crops.

13 A method of controlling insects and representatives of the order Acarina, which comprises contacting these pests, their various development stages or the locus thereof with a pesticidally effective amount of a compound of formula I according to any one of claims 1 to 8 or with a composition which contains a pesticidally effective amount of such a compound, together with adjuvants and carriers.

**Revendications pour les Etats Contractants**: BE, CH, DE, FR, GB, IT, LI, NL

1 - Composé de formule I

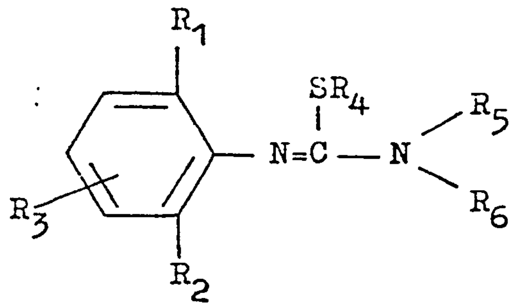

(I),

dans laquelle

$R_1$ représente un groupe alkyle en C 1-C 5;

$R_2$ représente l'hydrogène ou un groupe alkyle en-C 1-C 5;

$R_3$ représente l'hydrogène, un halogène, un groupe alkyle en C 1-C 5, phénoxy ou phénoxy substitué par 1 ou 2 atomes d'halogènes, groupes alkyle en C 1-C 5 ou trifluorométhyle;

$R_4$ représente un groupe alkyle en C 1-C 5, allyle ou propargyle;

$R_5$ représente un groupe alkyle en C 1-C 5, cycloalkyle en C 3-C 6, allyle ou propargyle; et

$R_6$ représente l'un des groupes $-CO-R_7$, $-CO-CO-R_8$, $-SO_2-R_7$ ou $-CO-OR_7$ dans lesquels

$R_7$ représente un groupe alkyle en C 1-C 5, phényle ou phényle substitué par 1 ou 2 atomes d'halogènes ou groupes méthyle, et

$R_8$ représente un groupe alcoxy en C 1-C 10 ou dialkylamino portant des groupes alkyle en C 1-C 5.

2 - Composé selon la revendication 1, de formule I dans laquelle

$R_1$ représente un groupe alkyle en C 1-C 4;

$R_2$ représente l'hydrogène ou un groupe alkyle en C 1-C 5;

$R_3$ représente l'hydrogène, le fluor, le chlore, un groupe alkyle en C 1-C 3, phénoxy ou phénoxy substitué par 1 ou 2 atomes de fluor ou de chlore, groupes méthyle, éthyle ou trifluorométhyle;

$R_4$ représente un groupe alkyle en C 1-C 3, allyle ou propargyle;

$R_5$ représente un groupe alkyle en C 2-C 5, cycloalkyle en C 3-C 6, allyle ou propargyle; et

$R_8$ représente l'un des groupes $-CO-R_7$, $-CO-CO-R_8$, $-SO_2-R_7$ ou $-CO-OR_7$ dans lesquels

$R_7$ représente un groupe alkyle en C 1-C 4, phényle ou phényle substitué par 1 ou 2 atomes de fluor ou de chlore ou groupes méthyle, et

$R_8$ représente un groupe alcoxy en C 1-C 4 ou dialkylamino portant des groupes alkyle en C 1-C 3.

3 - Composé selon la revendication 2, de formule I dans laquelle

$R_1$ représente un groupe alkyle en C 1-C 4;

$R_2$ représente un groupe alkyle en C 1-C 5;

$R_3$ représente l'hydrogène, le fluor, le chlore, un groupe méthyle, phénoxy, phénoxy substitué par un atome de fluor ou de chlore; un groupe méthyle ou trifluorométhyle;

$R_4$ représente un groupe alkyle en C 1-C 3, allyle ou propargyle;

$R_5$ représente un groupe alkyle en C 2-C 5, cyclopropyle, cyclohexyle, allyle ou propargyle; et

$R_6$ représente l'un des groupes $-CO-R_7$ ou $-CO-CO-R_8$ dans lesquels

$R_7$ représente un groupe méthyle, éthyle, phényle ou phényle substitué par 1 ou 2 atomes de fluor ou de chlore; et

$R_8$ représente un groupe méthoxy, éthoxy ou dialkylamino portant des groupes méthyle ou éthyle.

4 - Composé selon la revendication 3, de formule I dans laquelle

$R_1$ représente un groupe alkyle en C 1-C 3;

$R_2$ représente un groupe alkyle en C 1-C 5;

$R_3$ représente l'hydrogène ou un groupe 4-phénoxy;

$R_4$ représente un groupe alkyle en C 1-C 3, allyle ou propargyle;

$R_5$ représente un groupe alkyle en C 2-C 5;

$R_6$ représente l'un des groupes $-CO-R_7$ ou $-CO-CO-R_8$ dans lesquels

$R_7$ représente un groupe méthyle, éthyle, phényle ou difluorophényle, et

$R_8$ représente un groupe méthoxy, éthoxy, diméthylamino ou diéthylamino.

5 - Composé selon la revendication 4, caractérisé en ce que:

$R_1$ représente un groupe alkyle en C 1-C 3;

$R_2$ représente un groupe alkyle en C 2-C 4;

$R_3$ représente l'hydrogène ou un groupe 4-phénoxy;

$R_4$ représente un groupe méthyle;

$R_5$ représente un groupe alkyle en C 1-C 4, et

$R_6$ représente l'un des groupes $-CO-CH_3$ ou $-CO-CO-OC_2H_5$.

6 - Composé selon la revendication 5, de formule

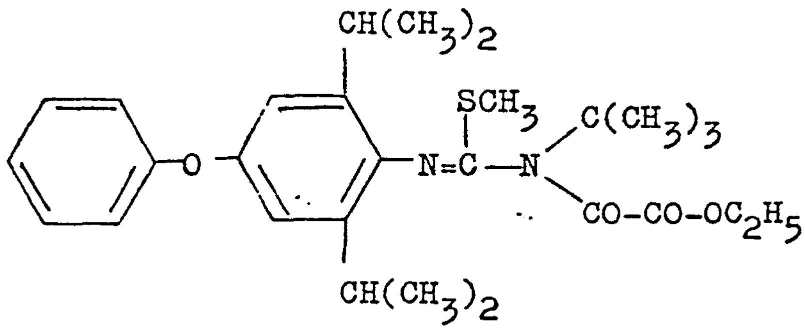

7 - Composé selon la revendication 5, de formule

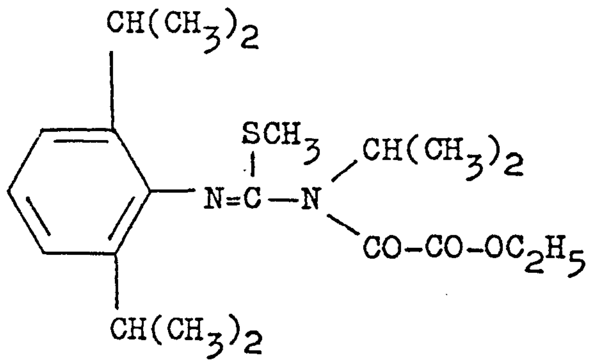

8 - Composé selon la revendication 5, de formule

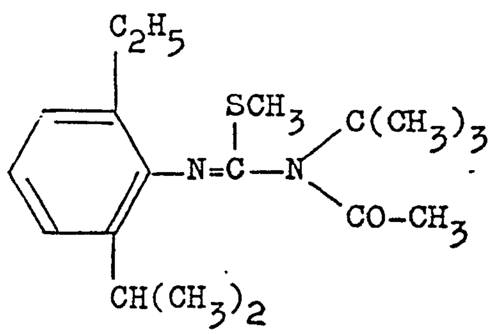

9 - Procédé de préparation d'un composé de formule I selon les revendications 1 à 8, caractérisé en ce que l'on fait réagir un composé de formule II

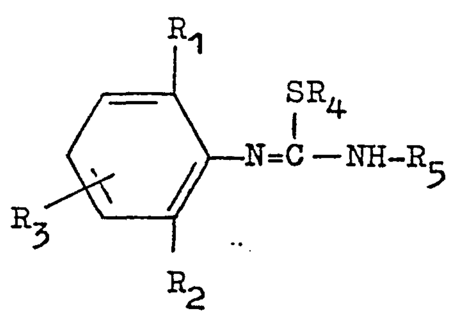

(II)

avec un composé de formule III

$X - R_6$ (III)

les symboles $R_1$ à $R_6$ ayant dans les formules II et III les significations indiquées dans les revendications 1 à 5 et X représentant un halogène, de préférence le chlore.

10 - Produit pour combattre les insectes et représentants de l'ordre des acariens nuisibles pour les végétaux, contenant en tant que composant actif un composé selon les revendications 1 à 8, avec des véhicules et/ou d'autres additifs appropriés.

11 - Utilisation d'un composé selon les revendications 1 à 8, dans la lutte contre les insectes et représentants de l'ordre des acariens nuisibles pour les végétaux.

12 - Utilisation selon la revendication 11, pour la lutte contre les cigales et insectes du sol nuisibles pour les végétaux.

13 - Utilisation selon la revendication 12, pour la lutte contre les cigales dans les cultures de riz.

14 - Procédé pour combattre les insectes et représentants de l'ordre des acariens, caractérisé en ce que l'on met en contact ou on traite les parasites ou leurs divers stades de développement ou leur habitat par une quantité pesticide efficace d'un composé de formule I selon l'une des revendications 1 à 8, ou par un produit contenant, avec des additifs et véhicules, une quantité pesticide efficace d'un tel composé.

**Revendications pour l'Etat Contractant**: AT

1 - Procédé de préparation d'un composé de formule I

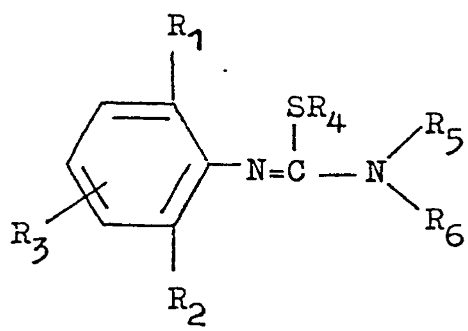

(I),

dans laquelle

$R_1$ représente un groupe alkyle en C 1-C 5;

$R_2$ représente l'hydrogène ou un groupe alkyle en C 1 -C 5;

$R_3$ représente l'hydrogène, un halogène, un groupe alkyle en C 1-C 5, phénoxy ou phénoxy substitué par 1 ou 2 atomes d'halogènes, groupes alkyle en C 1-C 5 ou trifluorométhyle;

$R_4$ représente un groupe alkyle en C 1-C 5, allyle ou propargyle;

$R_5$ représente un groupe alkyle en C 1-C 5, cycloalkyle en C 3-C 6, allyle ou propargyle; et

$R_6$ représente l'un des groupes $-CO-R_7$, $-CO-CO-R_8$, $-SO_2-R_7$ ou $-CO-OR_7$ dans lesquels

$R_7$ représente un groupe alkyle en C 1-C 5, phényle ou phényle substitué par 1 ou 2 atomes d'halogènes ou groupes méthyle, et

$R_8$ représente un groupe alcoxy en C 1-C 10 ou dialkylamino portant des groupes alkyle en C 1-C 5, caractérisé en ce que l'on fait réagir un composé de formule II

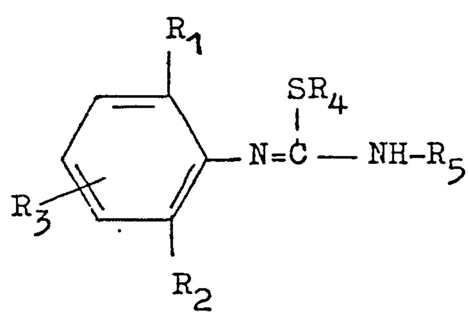

(II)

avec un composé de formule III

$X - R_6$ (III)

les symboles $R_1$ à $R_6$ ayant dans les formules II et III les significations indiquées ci-dessus et X représentant un halogène, de préférence le chlore.

2 - Procédé selon la revendication 1, pour la préparation d'un composé de formule I dans laquelle
$R_1$ représente un groupe alkyle en C 1-C 4;
$R_2$ représente l'hydrogène ou un groupe alkyle en C 1 -C 5;
$R_3$ représente l'hydrogène, le fluor, le chlore, un groupe alkyle en C 1-C 3, phénoxy ou phénoxy substitué par 1 ou 2 atomes de fluor ou de chlore, groupes méthyle, éthyle ou trifluorométhyle;
$R_4$ représente un groupe alkyle en C 1-C 3, allyle ou propargyle;
$R_5$ représente un groupe alkyle en C 2-C 5, cycloalkyle en C 3-C 6, allyle ou propargyle;
$R_6$ représente l'un des groupes $-CO-R_7$, $-CO-CO-R_8$, $-SO_2-R_7$ ou $-CO-OR_7$ dans lesquels
$R_7$ représente un groupe alkyle en C 1-C 4, phényle ou phényle substitué par 1 ou 2 atomes de fluor ou de chlore ou groupes méthyle; et
$R_8$ représente un groupe alcoxy en C 1-C 4 ou dialkylamino portant des groupes alkyle en C 1-C 3.

3 - Procédé selon la revendication 2, pour la préparation d'un composé de formule I dans laquelle
$R_1$ représente un groupe alkyle en C 1-C 4;
$R_2$ représente un groupe alkyle en C 1-C 5;
$R_3$ représente l'hydrogène, le fluor, le chlore, un groupe méthyle, phénoxy, phénoxy substitué par un atome de fluor ou de chlore, un groupe méthyle ou trifluorométhyle;
$R_4$ représente un groupe alkyle en C 1-C 3, allyle ou propargyle;
$R_5$ représente un groupe alkyle en C 2-C 5, cyclopropyle, cyclohexyle, allyle ou propargyle; et
$R_6$ représente l'un des groupes $-CO-R_7$ ou $-CO-CO-R_8$ dans lesquels
$R_7$ représente un groupe méthyle, éthyle, phényle ou phényle substitué par 1 ou 2 atomes de fluor ou de chlore; et
$R_8$ représente un groupe méthoxy, éthoxy ou dialkylamino portant des groupes méthyle ou éthyle.

4 - Procédé selon la revendication 3, pour la préparation d'un composé de formule I dans laquelle
$R_1$ représente un groupe alkyle en C 1-C 3;
$R_2$ représente un groupe alkyle en C 1-C 5;
$R_3$ représente l'hydrogène ou un groupe 4-phénoxy;
$R_4$ représente un groupe alkyle en C 1-C 3, allyle ou propargyle;
$R_5$ représente un groupe alkyle en C 2-C 5;
$R_6$ représente l'un des groupes $-CO-R_7$ ou $-CO-CO-R_8$ dans lesquels
$R_7$ représente un groupe méthyle, éthyle, phényle ou difluorophényle, et
$R_8$ représente un groupe méthoxy, éthoxy, diméthylamino ou diéthylamino.

5 - Procédé selon la revendication 4, pour la préparation d'un composé de formule I dans laquelle
$R_1$ représente un groupe alkyle en C 1-C 3;
$R_2$ représente un groupe alkyle en C 2-C 4;
$R_3$ représente l'hydrogène ou un groupe 4-phénoxy;
$R_4$ représente un groupe méthyle;
$R_5$ représente un groupe alkyle en C 1-C 4; et
$R_6$ représente l'un des groupes $-CO-CH_3$ ou $-CO-CO-OC_2H_5$.

6 - Procédé selon la revendication 5, pour la préparation du composé de formule

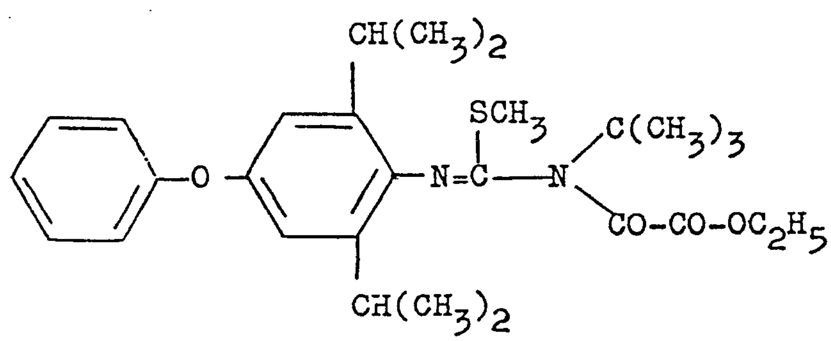

7 - Procédé selon la revendication 5, pour la préparation du composé de formule

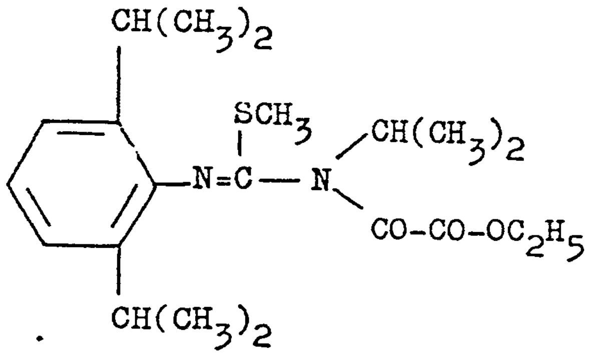

8 - Procédé selon la revendication 5, pour la préparation du composé de formule

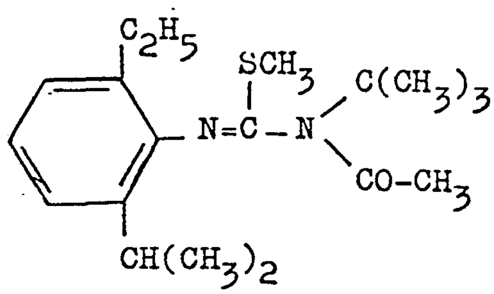

9 - Produit pour combattre les insectes et représentants de l'ordre des acariens nuisibles pour les végétaux, contenant en tant que composant actif un composé selon les revendications 1 à 8 avec des véhicules et/ou d'autres additifs appropriés.

10 - Utilisation d'un composé selon les revendications 1 à 8 dans la lutte contre les insectes et représentants de l'ordre des acariens nuisibles pour les végétaux.

11 - Utilisation selon la revendication 10, dans la lutte contre les cigales et insectes du sol nuisibles pour les végétaux.

12 - Utilisation selon la revendication 11, dans la lutte contre les cigales dans les cultures de riz.

13 - Procédé pour combattre les insectes et représentants de l'ordre des acariens, caractérisé en ce que l'on met en contact ou on traite les parasites ou leurs divers stades de développement ou leur habitat par une quantité pesticide efficace d'un composé de formule I selon l'une des revendications 1 à 8 ou par un produit contenant, avec des additifs et véhicules, une quantité pesticide efficace d'un tel composé.